# EUROPEAN PATENT APPLICATION

(11) **EP 4 458 828 A1**
(43) Date of publication of application: **06.11.2024**
(21) Application number: 23171778.6
(22) Date of filing: 05.05.2023
(51) Int. Cl.: C07D 491/04, B01D 61/02, B01J 31/40

(54) **A CONTINUOUS PROCESS FOR PRODUCING CHIRAL LACTONES**

(71) Applicant: DSM IP Assets B.V., 6221 BE Maastricht (NL)
(72) Inventor: MUELLER, Marc-André, 4303 Kaiseraugst (CH); GOY, Roman, 4303 Kaiseraugst (CH); BONRATH, Werner, 4303 Kaiseraugst (CH); MEDLOCK, Jonathan Alan, 4303 Kaiseraugst (CH)
(74) Representative: dsm-firmenich IP

(57) **Abstract**

The present invention provides a continuous process for producing chiral lactone compounds.

## Description

### Technical Field

The present invention is related to a continuous process for producing chiral lactones.

### Background of the Invention

Lactones are wildly useful as pharmaceutical or nutritional active substances or as intermediates for the manufacture of such active substances. Especially, (3aS, 6aR)-lactone is an important intermediate for the manufacture of biotin 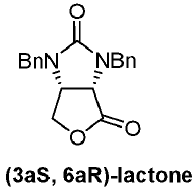 Bn is benzyl.

One efficient process for producing the (3aS, 6aR)-lactone intermediate comprises reacting a cyclic carboxylic acid anhydride with a chiral alcohol to obtain dicarboxylic acid monoesters, which are then reduced and further cyclized to obtain the lactone (see: WO2004094367A2).

An alternative process is asymmetric hydrogenation of a cyclic carboxylic acid anhydride with a chiral catalyst to directly obtain the chiral lactone in one step (see: Bonrath, W., Karge, R., Netscher, T., Roessler, F. and Spindler, F. Chiral Lactones by Asymmetric Hydrogenation - A Step Forward in (+)-Biotin Production, Asymmetric Catalysis on Industrial Scale (2010), edited by H.U. Blaser and H.J. Federsel)*.*

However, there is still need for a more efficient process.

### a. Description of the Figures

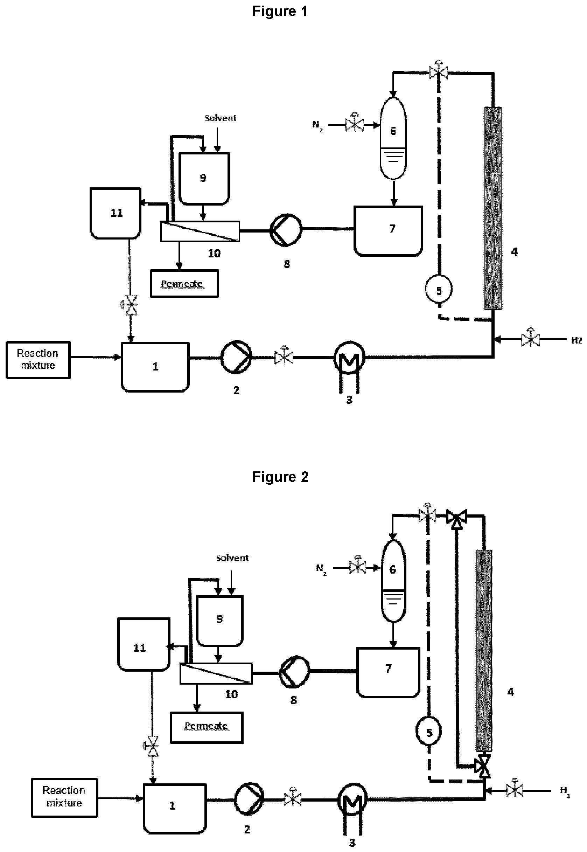

**Figure 1** indicates the working flow of one embodiment according to the present invention wherein the raw material of the compound of formula (II) is fully converted in a reactor 4, wherein 1: mixing tank, 2: high pressure pump, 3: heater, 4: reactor, 5: pressure regulator, 6: gas-liquid separator, 7: buffer tank, 8: high pressure pump, 9: buffer tank, 10: nanofiltration cell, 11: buffer tank; and
**Figure 2** indicates the working flow of another embodiment according to the present invention wherein the raw material of the compound of formula (II) is partially converted in reactor 4, wherein 1: mixing tank, 2: high pressure pump, 3: heater, 4: reactor, 5: pressure regulator, 6: gas-liquid separator, 7: buffer tank, 8: high pressure pump, 9: buffer tank, 10: nanofiltration cell, 11: buffer tank.

### Summary of the Invention

The present invention provides a continuous process for producing a lactone compound of formula (I) from a compound of formula (II), 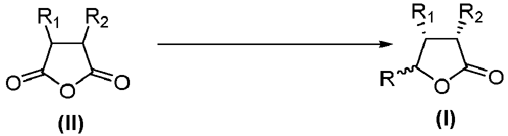
wherein R is H or hydroxy, and R₁ and R₂ are independently -NR₃R₄,
and R₃ and R₄ are independently H, alkyl, cycloalkyl, alkenyl, aryl, arylalkyl, arylalkenyl, cycloalkylalkyl, heterocyclyl, acyl, alkylsulphonyl, arylsulphonyl or a silyl group Si(alkyl)₃, Si(aryl)₃ or Si(alkyl)ₘ(aryl)ₙ, optionally substituted by one or more substituents, and m and n are independently 1 or 2;
or the two R₃ alternatively form together a carbonyl group and the two R₄ are independently defined as above.

### Detailed description of the Invention

In the present invention, the term "alkyl", used as such or as part of a group such as arylalkyl, cycloalkylalkyl, alkylsulphonyl and the silyl groups Si(alkyl)₃ and Si(alkyl)ₘ(aryl)ₙ, refers to both branched and straight-chain saturated aliphatic hydrocarbon groups having up to 12 carbon atoms, more preferably with up to 6 carbon atoms. Examples of the "alkyl" include but are not limited to methyl, ethyl, propyl, isopropyl, butyl, isobutyl, tert-butyl, pentyl, iso-pentyl, tert-pentyl, hexyl, isohexyl, tert-hexyl, octyl, isooctyl, tert-octyl, nonyl, isononyl, tert-nonyl, decyl, isodecyl and tert-decyl.

In the present invention, the term "cycloalkyl", used as such or as part of a group such as cycloalkylalkyl, refers to a monocyclic, bicyclic or spirocyclic saturated aliphatic hydrocarbon group having the specified number of carbon atoms. Examples of the "cycloalkyl" include but are not limited to cyclopropyl, methyl-cyclopropyl, 2,2-dimethyl-cyclobutyl, cyclopentyl, 2-ethyl-cyclopentyl, cyclohexyl, and so on.

In the present invention, the term "alkenyl", used as such or as part of a group such as arylalkenyl, refers to a non-aromatic hydrocarbon radical, straight, branched or cyclic, having up to 12 carbon atoms, more preferably with up to 6 carbon atoms, depending on the number of carbon atoms, containing up to three double bonds, preferably one double bond. Examples of the "alkenyl" is ethenyl, propenyl, allyl, butenyl, 2-methylbutenyl and cyclohexenyl.

In the present invention, the term "aryl", used as such or as part of a group such as arylalkyl, arylalkenyl, arylsulphonyl or the silyl groups Si(aryl)₃ and Si(alkyl)ₘ(aryl)ₙ, refers to a carbocyclic aromatic system containing one ring, or two or three rings fused together where in the ring atoms are all carbon. Example of the "aryl" include but are not limited to groups such as phenyl, benzyl, xylyl and naphthyl.

In the present invention, the term "heterocyclyl", used as such or as part of a group, refers to a non-aromatic saturated monocyclic, bicyclic, tricyclic or spirocyclic ring system comprising up to 7 atoms in each ring, or contains 3 to 14, or 5 to 10 ring atoms, in which one or more of the atoms in the ring system is an element other than carbon, for example, nitrogen, oxygen, phosphorus or sulfur, alone or in combination. Preferred heterocyclyl contain 4 to 7 ring atoms, more preferably 5 to 6 ring atoms. Non-limiting examples of suitable heterocyclyl rings include piperidyl, pyrrolidinyl, piperazinyl, morpholinyl, thiomorpholinyl, thiazolidinyl, 1,4-dioxanyl, tetrahydrofuranyl, tetrahy drothiophenyl, lactam, lactone, and the like.

In the present invention, the term "acyl", used as such or as part of groups, refers to a structure represented by R¹-C(=O)- wherein R¹ is alkyl or aryl as defined herein.

In the present invention, the term "alkoxyl", used as such or as part of a group, refers to the structure represented by (alkyl)-O-, wherein the alkyl is as defined herein.

In the present invention, the term "halo" or "halogen", used as such or as part of a group, refers to a group of elements including fluorine (F), chlorine (CI), bromine (Br) and iodine (I), preferably refers to Cl or Br, and accordingly a halide as used is the anion of a halogen.

In the present invention, the term "substituent" or "substituents" as used refers to C₁-C₆ alkyl, C₁-C₆ alkoxyl, C₁-C₆ alkylthio, aryl, hydroxyl, halo, halide, -NH₂, -NO₂, cyano and/or isocyano.

In the present invention, the symbol " 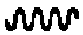 " as used in the compound formulas of the present invention means the connected group is connected to a chiral carbon in S- and/or R- configuration according to the Cahn-Ingold-Prelog priority rule.

In the present invention, the structure 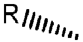 in the compound formulas of the present invention means the group R is oriented away from the viewer compared to chiral carbon it is connected to.

In the present invention, the structure 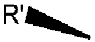 in the compound formulas of the present invention means the group R' is oriented closer to the viewer compared to chiral carbon it is connected to.

Particularly, the present invention provides a continuous process for producing a compound of formula (I) from a compound of formula (II), 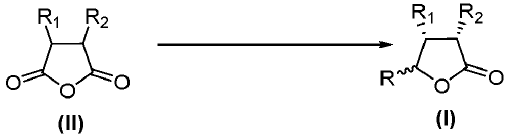 wherein R, R₁ and R₂ are each independently defined as above,
which comprises:
a) continuously feeding a reaction mixture containing the compound of formula (II) and hydrogen gas into a reactor;
b) asymmetrically hydrogenating the compound of formula (II) in the reactor to obtain a mixture containing the compound of formula (I);
c) proceeding nanofiltration on the mixture containing the compound of formula (I) to obtain a permeate and a retentate; and
d) collecting the compound of formula (I) from the permeate.

Preferably, R₁ and R₂ are independently -NR₃R₄, and the two R₃ form together a carbonyl group and the two R₄ are independently H, alkyl, cycloalkyl, alkenyl, aryl, arylalkyl, arylalkenyl, cycloalkylalkyl, heterocyclyl, acyl, alkylsulphonyl, arylsulphonyl or a silyl group Si(alkyl)₃, Si(aryl)₃ or Si(alkyl)ₘ(aryl)ₙ, optionally substituted by one or more substituents, and m and n are independently 1 or 2.

More preferably, the compound of formula (II) is the compound of formula (Ila),

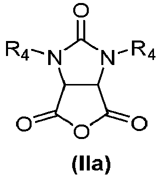

Wherein each of R₄ is dependently defined as above.

More preferably, the compound of formula (I) is the compound of formula (la):

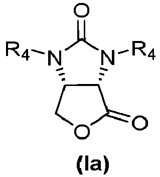

Wherein each of R₄ is dependently defined as above.

### Step a1: continuously feeding a reaction mixture containing the compound of formula (II) and hydrogen gas into a reactor

In the step a), the reaction mixture contains the starting material of the compound of formula (II) and other ingredients for asymmetric hydrogenation such as a solvent, a catalyst and optionally an additive.

In the step a), the solvent may be selected from the group consisting of hydrocarbons, chlorinated hydrocarbons, ethers and esters. Preferred solvents are selected from the group consisting of tetrahydrofuran (THF), dichloromethane (DCM), 2-methyltetrahydrofuran (Me-THF), cyclopentyl methyl ether (CPME), diethyl ether (Et₂O), ethyl acetate (EtOAc) and isopropyl acetate (iPrOAc). The solvent may be used in the step a) in an amount of from 1 mL to 20 mL, preferably from 5 mL to 15 mL such as 5, 6, 7, 8, 9 and 10 mL, per 1 mmol of the compound of formula (II).

In the step a), the catalyst is a chiral catalyst. Preferably, the chiral catalyst is a transition metal catalyst such as iridium (Ir) catalysts. More preferably, the chiral catalyst contains a chiral ligand such as bisphosphine ligands. Examples of the bisphosphine ligands include but are not limited to:
(R)-1-{(R_{P})-2-[2-(diphenylphosphino)phenyl]ferrocenyl}ethyldi(2-norbornyl)phosphine (SL-W022-1),
(S) -1-[(S)-1-[bis[3,5-bis(trifluoromethyl)phenyl]phosphino]ethyl]-2-[2-(diphenylphosphino)phenyl] ferrocene (SL-W001-2),
(R)-(+)-5,5'-bis-[di-(3,5-di-*tert*-butyl-4-methoxyphenyl)-phosphino]-4,4'-bi-1,3-benzodioxol ((R)-DTBM-SEGPHOS),
[(1R)-6,6'-dimethoxy[1,1'-biphenyl]-2,2'-diyl]bis[bis[3,5-bis(1 ,1-dimethylethyl)-4-methoxyphenyl]phosphine] ((R)-DTBM-MeOBiPHEP),
(+)-1,1'-Bis((2R,4R)-2,4-diethylphosphotano)ferrocene ((R,R)-ⁱPrXANTANE),
(S)-2,2'-Bis[bis(3,5-di-tert-butyl-4-methoxyphenyl)phosphino]-1,1'-binaphthyl ((S)-DTBM-BINAP), (S,S)-Et-FerroTANE, and/or
(S,S)-(R,R)-Ph-TRAP.

Preferably, the chiral catalyst contains a chiral ligand selected from the group consisting of (R)-DTBM-SEGPHOS, (R)-DTBM-MeOBiPHEP, (R,R)-ⁱPrXANTANE, (S,S)-Et-FerroTANE, and/or (S,S)-(R,R)-Ph-TRAP, more preferably, (R)-DTBM-SEGPHOS and/or (R)-DTBM-MeOBiPHEP.

In the step a), the chiral catalyst may be prepared from a metal catalyst precursor and a chiral ligand according to a method known in the art. The metal catalyst precursor may be selected from the group consisting of Ir catalyst precursors including but not limited to [Ir(COD)Cl]₂, [Ir(NBD)Cl]₂, [Ir(CH₂CH₂)₂Cl]₂, [Ir(COD)₂]Y or [Ir(NBD)₂]Y (COD is 1,5-cyclooctadiene and NBD is 2,5-norbonadiene), wherein Y is an anion such as halogen anion, BF₄⁻, B(Ar)₄⁻ (for example, BARF(i.e., Tetrakis[3,5-bis(trifluoromethyl)phenyl]borate)), ClO₄⁻, SbF₆⁻, PF₆⁻ and/or CF₃SO₃⁻. The chiral ligand may be selected from the group consisting of (R)-DTBM-SEGPHOS, (R)-DTBM-MeOBiPHEP, (R,R)-ⁱPrXANTANE, (S,S)-Et-FerroTANE, and/or (S,S)-(R,R)-Ph-TRAP.

Preferable examples of the chiral catalyst used in the step a) of the present invention include but are not limited to Ir catalysts such as the complexes formed by a metal precursor [Ir(COD)Cl]₂ and a ligand (R)-DTBM-SEGPHOS or (R)-DTBM-MeOBiPHEP.

The chiral catalyst in the step a) of the present invention may be used after isolation or as prepared in-situ. Preferably, the catalyst is generated in-situ.

In the process of the present invention, the mole ratio (S/C) between the compound of formula (II) and the chiral catalyst is in the range of from 50:1 to 1,000,000:1, preferably from 100:1 to 500,000: 1, more preferably from 500:1 to 100,000: 1 and the most preferably from 1,000:1 to 50,000:1 such as 1,000:1, 2,000:1, 3,000:1, 4,000:1, 5,000:1, 6,000:1, 7,000:1, 8,000:1, 9,000:1, 10,000:1, 15,000:1 and 20,000:1.

In the step a), the additive may be any iodine-containing reagent such as iodine (I₂) or any iodide or mixture thereof. The iodide may be selected from the group consisting of hydroiodic acid, iodic acid, iodine monochloride, cyanogen iodide, silicon tetraiodide, carbon tetraiodide, iodine pentoxide, iodide salts such as lithium iodide, sodium iodide, potassium iodide, calcium iodide, cupper iodide, cadmium iodide and tetra-n-butylammonium iodide (Bu₄NI), metaperiodates such as sodium metaperiodate, alkyl iodides such as methyl iodide and iodoform, and mixture thereof. Preferably, the additive is iodine or Bu₄NI. The additive may be used in an amount of from 0.1 mol to 10 mol, more preferably from 0.2 mol to 8 mol, the most preferably from 0.5 mol to 5 mole such as 0.5 mol and 4 mol, per 1 mol of the metal contained in the chiral catalyst used in the process of the present invention.

Preferably, the iodine-containing reagent in the process of the present invention is used in the presence of an acid. The acid may be any acid know in the art, including but not limited to inorganic acids such as hydrochloric acid (HCl) and organic acids such as acetic acid (AcOH). Preferably the acid is AcOH. The acid may be used in an amount of from 0.1 mol to 10 mol, more preferably from 0.5 mol to 5 mol such as 1 mol, per 1 mol of the iodine-containing reagent used in the process of the present invention.

In the step a), the reaction mixture may be warmed to the reaction temperature before feeding into the reactor. preferably, the reaction mixture may be warmed to 20°C to 120°C such as 100°C.

The reaction mixture in the step a) may be prepared in a mixing tank and preferably after warmed to the reaction temperature, pumped into the reactor.

In the step a), hydrogen gas is fed together with the reaction mixture into the reactor. Preferably, the hydrogen gas is fed under pressure of from 10 bar to 100 bar, more preferably under the pressure of from 20 bar to 80 bar such as 30, 40 50, 60, 70 and 80 bar.

In the step a), the reactor may be any reactor suitable for asymmetrical hydrogenation of the compound of formula (II) which is known in the art. Examples of the reactor are continuous stirred-tank reactor (CSTR), a cascade of CSTRs and a flow reactor such as the reactor disclosed in international patent publication WO2018/202640 A1.

### Step b): asymmetrically hydrogenating the compound of formula (II) in the reactor to obtain a mixture containing the compound of formula (I)

In the step b), the asymmetrical hydrogenation of the compound of formula (II) may be carried out under the condition known in the art, for example, as disclosed in : Bonrath, W., Karge, R., Netscher, T., Roessler, F. and Spindler, F. Chiral Lactones by Asymmetric Hydrogenation - A Step Forward in (+)-Biotin Production, Asymmetric Catalysis on Industrial Scale (2010), edited by H.U. Blaser and H.J. Federsel*.*

Preferably, the asymmetrical hydrogenation of the compound of formula (II) in the step b) is caried out at from 0°C to 150°C, preferably from 20°C to 120°C such as 100°C, under the pressure of from 1 bar to 150 bar, preferably 10 bar to 100 bar, such as 30 bar.

Preferably, in the step b), the reaction mixture passes a CSTR reactor to achieve the asymmetrical hydrogenation of the compound of formula (II). As anticipated by any person skilled in the art, the length and diameter of the reactor can be designed to achieve full conversion of the starting material of the compound of formula (II) at the exit of the reactor.

In some embodiment, the compound of formula (II) is fully converted in the reactor. In other embodiment, the compound of formula (II) is partially converted in the reactor.

In the embodiment that the compound of formula (II) is partially converted in the reactor, the obtained reaction mixture is preferably fed back into the reactor one or more times till the compound of formula (II) is fully converted.

According to the step b), a mixture containing the compound of formula (I) is obtained.

### Step c1: proceeding nanofiltration on the mixture containing the compound of formula (I) to obtain a permeate and a retentate

In the step c), the nanofiltration is conducted with nanofiltration membranes which have pore sizes of from 0.5 nm to 10 nm. Preferably, the nanofiltration membranes are selected from polymeric membranes and ceramic membranes. More preferably, the nanofiltration membranes are polymeric.

In the step c), the nanofiltration is carried out in an organic solvent. The organic solvent may be selected from the group consisting of hydrocarbons, chlorinated hydrocarbons, ethers and esters, preferably tetrahydrofuran (THF), dichloromethane (DCM), 2-methyltetrahydrofuran (Me-THF), cyclopentyl methyl ether (CPME), diethyl ether (Et₂O), ethyl acetate (EtOAc) and isopropyl acetate (iPrOAc). The organic solvent is used in an amount of from 0.1 diavolumes (DV) to 4.0 DV, preferably from 0.5 DV to 3 DV of the mixture to be treated.

In the step c), the nanofiltration may be carried out at the temperature of from 10°C to 100°C, preferably from 15°C to 70°C.

In the step c), a metal coordinating compound is preferable added into the mixture containing the compound of formula (I) before the nanofiltration. The metal coordinating compound can be any which is known in the art suitable to coordinate a metal of the catalyst used in the process of the present invention in a monodentate or chelating mode. Examples of the metal coordinating compound include but are not limited to 1,5-cyclooctadiiene (COD), bicyclo[2.2.1]hepta-2,5-dienyl (NBD), cyclooctene (COE) and ethylene. The metal coordinating compound may be added in an amount of from 1 mol to 10 moles, preferably from 2 moles to 8 moles such as 6 mols and 7 mols, per 1 mol of the metal of the chiral catalyst used in the process of the present invention.

In the step c), the mixture containing the compound of formula (I) may optionally be subjected to a pre-treatment before the nanofiltration. The pre-treatment may be any treatment which is beneficial to the nanofiltration of the mixture. As an example, the mixture containing the compound of formula (I) is concentrated before the nanofiltration. As another example, the mixture containing the compound of formula (I) is separated from the hydrogen gas before the nanofiltration. As a further example, the mixture containing the compound of formula (I) is purged with nitrogen to remove hydrogen gas contained in the mixture before the nanofiltration. As understand by any person skilled in the art, the treatments can be used alone or in combination.

Preferably, in the step c), the mixture containing the compound of formula (I) is separated from the hydrogen gas. More preferably, the mixture containing the compound of formula (I) is separated from the hydrogen gas in a gas-liquid separator and then purged with nitrogen before nanofiltration.

According to the step c), a permeate containing the compound of formula (I) and a retentate containing the organic solvent and the catalyst are obtained.

### Step d): collecting the compound of formula (I) from the permeate

In the step d), the compound of formula (I) is collected from the permeate by a process known in the art, such as diafiltration.

Meanwhile, in the step d), the retentate contains the catalyst and solvent. It may be recycled into the reaction mixture in the step a) directly or after separation or purification and/or reactivation of the catalyst. Preferably, the retentate is recycled into the reaction mixture in the step a) directly.

In an embodiment of the present invention, referring to Figure 1, the reaction mixture is prepared in a mixing tank **1** by adding the solvent, the starting material, the catalyst (prepared from the precursor and the ligand) and the optional additive as described above. This mixture is pumped via a high pressure pump **2** through a heater **3** (e.g., cartridge-heater) to bring the reaction mixture to said reaction temperature e.g., 100°C. In the flow, hydrogen gas (H₂) is fed, and the specific pressure is set up to e.g., 70 bar, regulated by the high-pressure pump **2.** The reaction mixture passes a reactor **4,** which is designed in length and diameter to achieve full conversion of starting material at the exit of the reactor **4.** A back pressure regulator **5** is located at the downstream of the reactor to control the pressure at the reactor inlet. The reaction mixture flows into a gas-liquid separator **6,** where the pressure is released, and the mixture is purged with nitrogen (N₂). The liquid phase containing the product, the catalyst and the solvent is flowing into a buffer tank **7,** where optionally a metal coordinating compound such as COD is added. From there, a high pressure pump **8** (which sets up the target pressure for nanofiltration *e.g.*, 20 bar) pumps the mixture into the nanofiltration cells **10.** There, organic solvent is continuously fed from a buffer tank **9** and the product permeates through the membrane, whereas the catalyst is hold back in the retentate and flows into another buffer tank **11.** The product is separated by >95% by diafiltration (by continuously adding fresh solvent into the buffer tank **9** for cross flow). Once the product is separated, the catalyst solution (retentate) with < 5% product is then recycled, optionally after catalyst reactivation, back from the buffer tank **11** into the mixing tank **1.** New starting material (and optionally fresh solvent, catalyst, and additive) is added, and the reaction cycle starts again.

In another embodiment of the present invention, referring to Figure 2, The reaction mixture is prepared in a mixing tank 1 by adding the solvent, the starting material, the catalyst (prepared from the precursor and the ligand) and the optional additive as described above. This mixture is pumped via a high pressure pump **2** through a heater **3** (e.g., cartridge-heater) to bring the reaction mixture to said reaction temperature e.g., 100°C. In an upward flow, hydrogen gas (H₂) is fed, and the specific pressure is set up to e.g., 70 bar, regulated by the high-pressure pump **2.** The reaction mixture passes a reactor **4,** which is designed in length and diameter to achieve partial conversion of starting material by 1-10% at the exit of the reactor **4.** A back pressure regulator **5** is located at the downstream of the reactor to control the pressure at the reactor inlet. The reaction mixture is circulated within a loop through the reactor **4** until a conversion of >99.9 % of the starting material. Afterwards the stream is split in a feed-and-bleed mode, whereas 80-99 % of the stream circulated in the loop through the reactor **4** and 1-10% of stream flows into a gas-liquid separator **6,** where the pressure is released, and the mixture is purged with nitrogen (N₂). The liquid phase containing the product, the catalyst and the solvent is flowing into a buffer tank **7,** where optionally a metal coordinating compound such as COD is added. From there a high pressure pump **8** (which sets up the target pressure for nanofiltration e.g., 20 bar) pumps the mixture into nanofiltration cells **10.** There, organic solvent is continuously fed from a buffer tank **9** and the product permeates through the membrane, whereas the catalyst is hold back in the retentate and flows into another buffer tank **11.** The product is separated by >95% by diafiltration (by continuously adding fresh solvent into the buffer tank **9** for cross flow). The retentate with < 5% product is then recycled, optionally after catalyst reactivation, back from the buffer tank **11** into the mixing tank **1.** New starting material (and optionally fresh solvent, catalyst and additive) is added, and the reaction cycle starts again.

The process of the present invention provides the compound of formula (I) in a continuous way with improved yield and selectivity and thus improves the efficiency.

The process of the present invention is further illustrated by the following examples.

### Examples

### Example 1

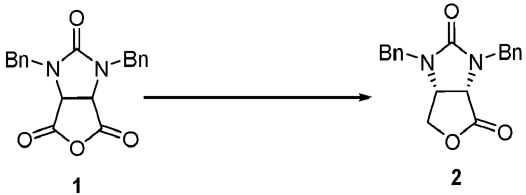

Referring to figure 1, the reaction mixture was prepared in the mixing tank **1** by adding Me-THF (1,500 mL), compound **1** (76.22 g, 0.23 mol), [IrCODCl]₂ (15.11 mg, 22.5 µmmol), DTBM-SEGPHOS (1 equivalent with respect to iridium) and iodine (5.71 mg, 22.5 µmmol). The mixture was pumped via the high pressure pump **2** through the heater **3** with a velocity of 70 kg/h to reach the desired reaction temperature of 120°C. After stabilization of reaction temperature and pressure (30 bar), the liquid mass flowrate was regulated to 20 kg/h and pure hydrogen was supplied from an external source in an upward flow feed. The reaction mixture passed the CSTR **4,** which was designed in length and diameter to achieve full conversion of starting material at the exit of the CSTR **4.** A back pressure regulator **5** was located at the downstream of the reactor to control the pressure at the reactor inlet. The off-gas was separated from the liquid and cooled down in the gas-liquid separator **6** and the mixture was purged with nitrogen. The liquid phase flowed into the buffer tank **7,** where COD (6 equivalents with respect to iridium) was added. From there, a high-pressure pump **8** (which sets up the target pressure for the nanofiltration) pumped the mixture into the nanofiltration (OSN) cells **10** and Me-THF was also continuously fed from the buffer tank **9.** The obtained reaction mixture was subjected during 4 hours in a diafiltration crossflow mode to a nanofiltration using a DuraMem 500 membrane (MWCO = ca. 500 Dalton, Evonik Industries AG, Germany) having a surface area of 42 cm² arranged in the experimental layout. A pressure of 15 bar, the temperature of 25 °C and a diafiltration feed of 5 mL/min Me-THF were applied. A 20 mL sample was taken from the feed material, the permeate and the retentate, respectively to determine the amount of Iridium by inductively coupled plasma and the desired compound **2** (yield and/or e.e.) by HPLC:
- Feed material of OSN: 8.647 mg Iridium, 74.79 g compound 2, 99.0% yield, 95 e.e.%
- Permeate of OSN: 0.432 mg Iridium, 72.53 g compound **2**
- Retentate of OSN: 8,152 mg Iridium, 2.12 g compound **2**

The product was separated by -97% efficiency into permeate, whereas ~5 % of Iridium were lost into permeate. The retentate contained the main part of the catalyst (95%) with < 3% of compound 2. The retentate was then recycled into the mixing tank 1. New compound 1 (and optionally fresh solvent, catalyst, and additive) was continuously added, and the reaction cycle started again.

## Claims

1. A continuous process for producing a compound of formula (I) from a compound of formula (II), 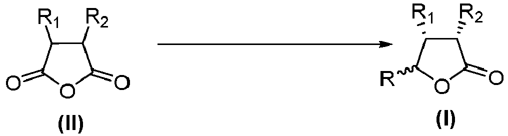
wherein R is H or hydroxy, and R₁ and R₂ are independently -NR₃R₄,
and R₃ and R₄ are independently H, alkyl, cycloalkyl, alkenyl, aryl, arylalkyl, arylalkenyl, cycloalkylalkyl, heterocyclyl, acyl, alkylsulphonyl, arylsulphonyl or a silyl group Si(alkyl)₃, Si(aryl)₃ or Si(alkyl)ₘ(aryl)ₙ, optionally substituted by one or more substituents, and m and n are independently 1 or 2;
or the two R₃ alternatively form together a carbonyl group and the two R₄ are independently defined as above,
which comprises:
a) continuously feeding a reaction mixture containing the compound of formula (II) and hydrogen gas into a reactor;
b) asymmetrically hydrogenating the compound of formula (II) in the reactor to obtain a mixture containing the compound of formula (I);
c) proceeding nanofiltration on the mixture containing the compound of formula (I) to obtain a permeate and a retentate; and
d) collecting the compound of formula (I) from the permeate.

2. The process of claim 1, wherein R₁ and R₂ are independently -NR₃R₄, and the two R₃ form together a carbonyl group, and the two R₄ are independently H, alkyl, cycloalkyl, alkenyl, aryl, arylalkyl, arylalkenyl, cycloalkylalkyl, heterocyclyl, acyl, alkylsulphonyl, arylsulphonyl or a silyl group Si(alkyl)₃, Si(aryl)₃ or Si(alkyl)ₘ(aryl)ₙ, optionally substituted by one or more substituents, and m and n are independently 1 or 2.

3. The process of claim 1 or 2, wherein in the step a) the reaction mixture further contains a solvent, a catalyst and optionally an additive.

4. The process of claim 3, wherein the solvent is selected from the group consisting of tetrahydrofuran (THF), dichloromethane (DCM), 2-methyltetrahydrofuran (Me-THF), cyclopentyl methyl ether (CPME), diethyl ether (Et₂O), ethyl acetate (EtOAc) and isopropyl acetate (iPrOAc).

5. The process of claim 3, wherein the catalyst is a chiral catalyst, preferably a transition metal catalyst, more preferably an iridium (Ir) catalyst.

6. The process of claim 3 or 5, wherein the catalyst contains a chiral ligand selected from the group consisting of:
(R)-1-{(R_{P})-2-[2-(diphenylphosphino)phenyl]ferrocenyl}ethyldi(2-norbornyl)phosphine (SL-W022-1),
(S)-1-[(S)-1-[bis[3,5-bis(trifluoromethyl)phenyl]phosphino]ethyl]-2-[2-(diphenylphosphino)phenyl] ferrocene (SL-W001-2),
(R)-(+)-5,5'-bis-[di-(3,5-di-*tert*-butyl-4-methoxyphenyl)-phosphino]-4,4'-bi-1,3-benzodioxol ((R)-DTBM-SEGPHOS),
[(1R)-6,6'-dimethoxy[1,1'-biphenyl]-2,2'-diyl]bis[bis[3,5-bis(1,1-dimethylethyl)-4-methoxyphenyl]phosphine] ((R)-DTBM-MeOBiPHEP),
(+)-1,1'-Bis((2R,4R)-2,4-diethylphosphotano)ferrocene ((R,R)-ⁱPrXANTANE),
(S)-2,2'-Bis[bis(3,5-di-tert-butyl-4-methoxyphenyl)phosphino]-1,1'-binaphthyl ((S)-DTBM-BINAP),
(S,S)-Et-FerroTANE, and/or
(S,S)-(R,R)-Ph-TRAP.

7. The process of claim 3 or 5, wherein the catalyst is a complex formed by a metal precursor [Ir(COD)Cl]₂ and a ligand (R)-DTBM-SEGPHOS or (R)-DTBM-MeOBiPHEP.

8. The process of claim 3, wherein the additive is an iodine-containing reagent, preferably is iodine (I₂) or an iodide or mixture thereof.

9. The process of claim 1 or 2, wherein in the step a) the reaction mixture is warmed to 20°C to 120°C before feeding into the reactor.

10. The process of claim 1 or 2, wherein in the step a) the reactor is a continuous stirred-tank reactor (CSTR), a cascade o CSTRs or a flow reactor.

11. The process of claim 1 or 2, wherein in the step b) the compound of formula (II) is fully converted in the reactor.

12. The process of claim 1 or 2, wherein in the step b) the compound of formula (II) is partially converted in the reactor and the obtained reaction mixture is fed back into the reactor.

13. The process of claim 1 or 2, wherein in the step c) the nanofiltration is conducted with nanofiltration membranes which have pore sizes of from 0.5 nm to 10 nm.

14. The process of claim 13, wherein the nanofiltration membranes are selected from the group consisting of polymeric membranes and ceramic membranes.

15. The process of claim 1 or 2, wherein in the step c) the nanofiltration is carried out in an organic solvent selected from the group consisting of tetrahydrofuran (THF), dichloromethane (DCM), 2-methyltetrahydrofuran (Me-THF), cyclopentyl methyl ether (CPME), diethyl ether (Et₂O), ethyl acetate (EtOAc) and isopropyl acetate (iPrOAc).

16. The process of claim 1 or 2, wherein in the step c) the nanofiltration is carried out at the temperature of from 10°C to 100°C, preferably from 15°C to 70°C.

17. The process of claim 1 or 2, wherein in the step c) a metal coordinating compound is added into the mixture containing the compound of formula (I) before the nanofiltration.

18. The process of claim 17, wherein the metal coordinating compound is selected from the group consisting of 1,5-cyclooctadiiene (COD), bicyclo[2.2.1]hepta-2,5-dienyl(NBD), cyclooctene (COE) and ethylene.

19. The process of claim 1 or 2, wherein in the step c) the mixture containing the compound of formula (I) is separated from the hydrogen gas before the nanofiltration.

20. The process of claim 1 or 2, wherein in the step d) the retentate is recycled into the reaction mixture in the step a).
